# EUROPEAN PATENT APPLICATION

(11) **EP 4 721 641 A1**
(43) Date of publication of application: **08.04.2026**
(21) Application number: 25207129.5
(22) Date of filing: 07.10.2025
(51) Int. Cl.: A61B 1/00, A61B 90/57

(54) **SURGICAL EQUIPMENT HOLDER**

(30) Priority: 07.10.2024 US 202463704276 P; 05.10.2025 US 202519350055
(71) Applicant: LSI Solutions, Inc., Victor, NY 14564 (US)
(72) Inventor: SAUER, Jude S., Pittsford, NY 14534 (US)
(74) Representative: Flügel Preissner Schober Seidel

(57) **Abstract**

A scope clutch assembly (10) includes a clutch member (14) having a hub portion (26), and a plurality of elongated distal engagement members (42) extend from a distal end of the hub portion (26). The clutch member (14) also includes a plurality of elongated proximal engagement members (142) that extend from a proximal end of the hub portion (26). Each of the distal engagement members (42) and the proximal engagement members (142) have portions that are configured to selectively engage corresponding portions of an exterior surface of a scope (66) that is inserted through an interior portion of the clutch member (14) to prevent the scope (66) from displacing relative to the clutch member (14) and/or to prevent/limit movement of the scope (66) relative to the clutch member (14). An adjustment member (12) displacably coupled to the clutch member (14) controls the degree of engagement between the portions of the proximal engagement members (142) engaging corresponding portions of the exterior surface of the scope (66).

## Description

### CROSS-REFERENCE TO RELATED APPLICATIONS

This patent application claims priority to U.S. Provisional Patent Application No. 63/704,276, filed October 7, 2024, and U.S. Patent Application No. 19/350,055, filed October 5, 2025, which are incorporated by reference herein in their entirety.

### FIELD OF THE INVENTION

The claimed invention relates to surgical devices, and more specifically to an adjustable holder for surgical equipment.

### BACKGROUND OF THE INVENTION

Minimally invasive surgery ("MIS") has transformed modern medical practices by enabling procedures through small incisions, thereby reducing patient trauma, shortening recovery times, and lowering the risk of postoperative complications compared to traditional open surgeries. During MIS procedures, multiple instruments may be introduced through an incision, and one of the instruments may be an endoscope, a flexible or rigid tube equipped with a camera and light source that provides surgeons with high-resolution, real-time visualization of internal organs and tissues. However, the precise placement and stabilization of the endoscope during procedures remain critical challenges, as unintended movements - caused by patient respiration, surgeon adjustments, or external vibrations - can obscure the surgical field, increase procedural complexity, and potentially compromise outcomes. These issues underscore the need for an advanced positioning mechanisms for scopes having differing diameters that ensures stability and has a streamlined shape that maximizes access to the surgical site.

### BRIEF SUMMARY OF THE INVENTION

The invention is set out in the appended claims. A clutch assembly comprises a clutch member extending along a clutch axis from a proximal end to a distal end, the clutch member having one or more interior surfaces that define an interior portion, the clutch member comprising a hub portion extending from a proximal end to a distal end, the proximal end of the hub portion being offset from the proximal end of the clutch member along the clutch axis by a first distance1, and the distal end of the hub portion may be offset from the distal end of the clutch member (along the clutch axis) by a second distance, the hub portion having an external threaded portion formed on a portion of an exterior surface of the hub portion. The clutch member also includes a plurality of elongated distal engagement members, each of the plurality of distal engagement members extending from a proximal end to a distal end, the proximal end of each of the plurality of distal engagement members extending from a corresponding portion of the distal end of the hub portion , and the distal end of at least one of the plurality of distal engagement members being disposed at the distal end of the clutch member. The clutch member further includes a plurality of elongated proximal engagement members, each of the plurality of proximal engagement members extending from a proximal end to a distal end, the distal end of each of the plurality of proximal engagement members extending from a corresponding portion of the proximal end of the hub portion, and the proximal end of at least one of the plurality of proximal engagement members being disposed at the proximal end of the clutch member, wherein a proximal engagement protrusion is disposed on or along a portion of an exterior surface of each of the plurality of proximal engagement members, and wherein each of the proximal engagement protrusions 64a extends radially outward from the clutch axis.

The clutch assembly includes an adjustment member coupled to the clutch member, the adjustment member extending from a proximal end to a distal end along a member axis that may be coaxially aligned with the clutch axis, the adjustment member having a central aperture extending along the member axis, and the central aperture being defined by one or more internal surfaces, with a first portion of the one or more internal surfaces including a threaded portion that is configured to engage the external threaded portion formed on the exterior surface of the hub portion such that the adjustment member is displaceable relative to the clutch member, and with a second portion of the one or more internal surfaces defining a circumferential ridge portion, wherein a portion of a contact surface of the ridge portion is configured to engage a corresponding contact surface of one of the proximal engagement protrusions such that (a) when the adjustment member is displaced in a first direction relative to the clutch member along the member axis, the proximal end of each of the proximal engagement protrusions is displaced towards the member axis to increase engagement between the proximal engagement protrusions and corresponding portions of an exterior surface of a scope such that displacement of the scope relative to the clutch member is prevented; and (b) when the adjustment member is displaced in a second direction relative to the clutch member along the member axis, the proximal end of each of the proximal engagement protrusions is displaced away from the member axis to decrease engagement between the proximal engagement protrusions and the corresponding portions of the exterior surface of the scope such that displacement of the scope relative to the clutch member is allowed.

### BRIEF DESCRIPTION OF THE DRAWINGS

Figs. 1A to 1E are various views of an embodiment of a scope clutch assembly;
Fig. 2A is a cross-sectional view of the embodiment of the scope clutch assembly taken along section line 2A-2A of Fig. 1A;
Fig. 5 is a detailed view of a first engagement slot 43 of the clutch member;
Fig. 6 is a detailed view of a first distal engagement member 42a of the clutch member;
Fig 7A is a cross-sectional view of engagement protrusions of the distal engagement members of the clutch member engaging portions of an external surface of a first scope having a first diameter;
Fig 7B is a cross-sectional view of engagement protrusions of the distal engagement members of the clutch member engaging portions of an external surface of a second scope having a second diameter;
Fig. 8 is a detailed view of a first engagement slot of the proximal engagement members of the clutch member;
Fig 9 is a cross-sectional view of the proximal engagement members of the clutch member engaging portions of an external surface of a scope; and
Fig. 10 is a perspective view of the embodiment of the scope clutch assembly with a scope inserted.

### DETAILED DESCRIPTION

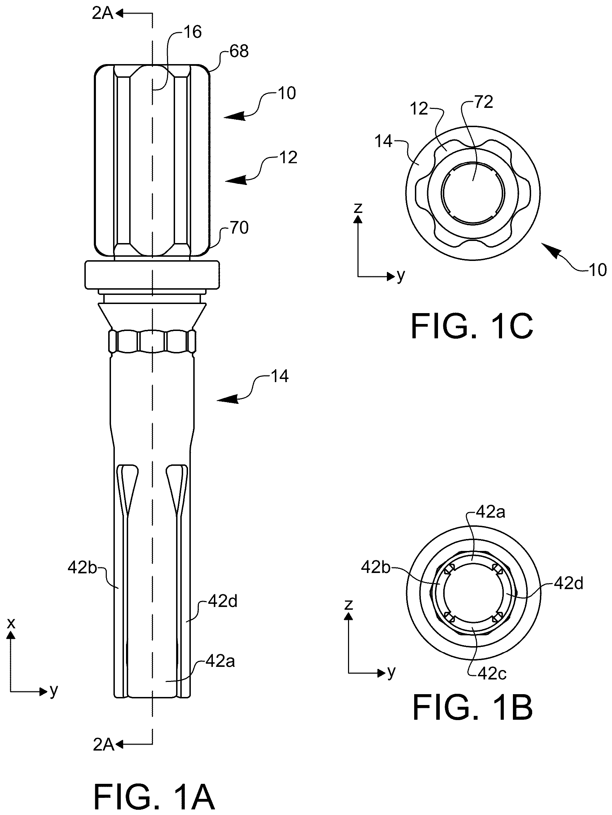

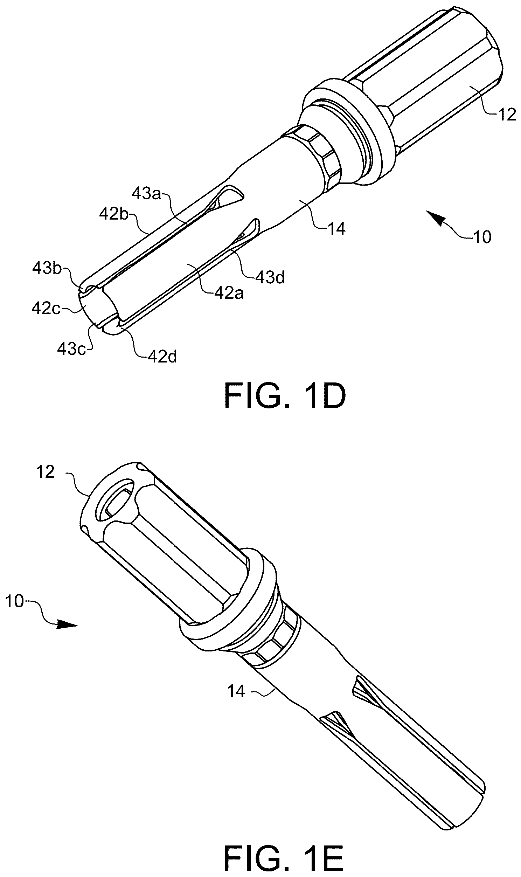

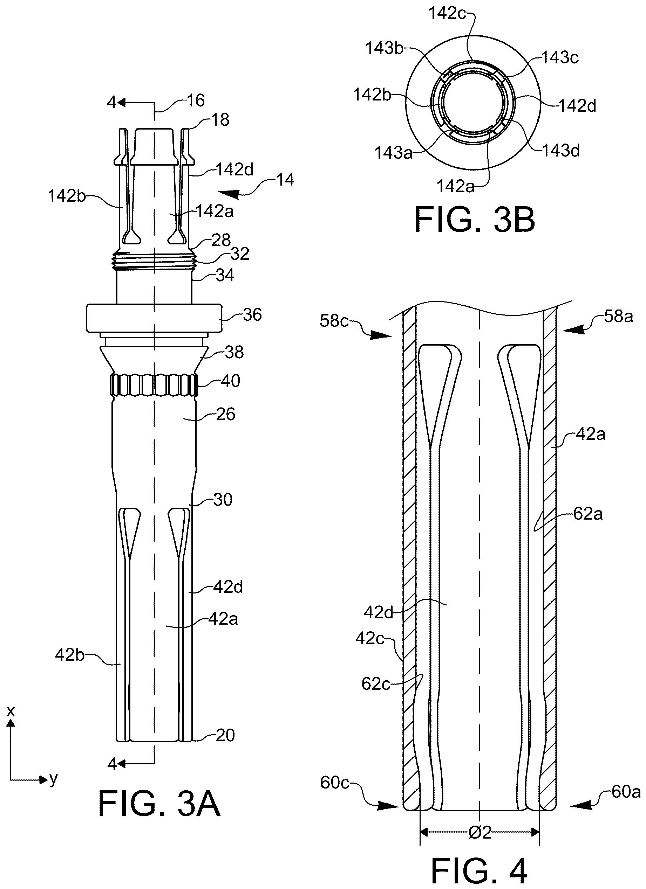

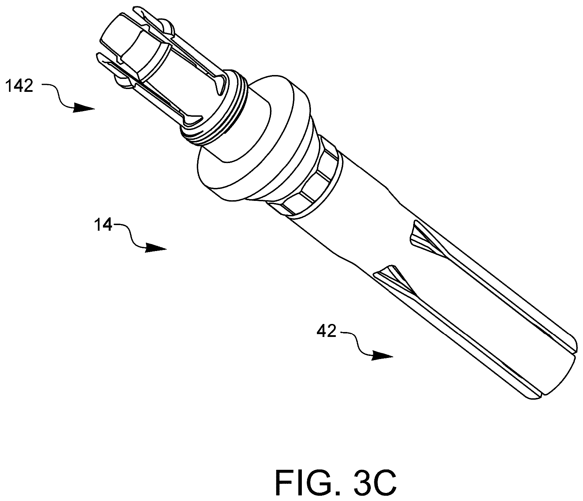

Figures 1A to 1E illustrate various an embodiment of a scope clutch assembly 10 that includes an adjustment member 12 displaceably coupled to a clutch member 14. As illustrated in Fig. 3A to 3C, the clutch member 14 is elongated and substantially cylindrical and extends along a clutch axis 16 from a proximal end 18 to a distal end 20, and the clutch axis 16 may be parallel to the X-axis of the reference coordinate system of Fig 1A. The clutch member 14 may include a hub portion 26 that extends from a proximal end 28 to a distal end 30, and a plurality of elongated distal engagement members 42 may extend from the distal end 30 of the hub portion 26. The clutch member 14 may also include a plurality of elongated proximal engagement members 142 that may extend from the proximal end 28 of the hub portion 26. Each of the distal engagement members 42 and the proximal engagement members 142 may have portions that are configured to selectively engage corresponding portions of an exterior surface of a scope 66, such as an endoscope, that is inserted through an interior portion 24 of the clutch member 14, to prevent the scope 66 from displacing along the clutch axis 16 relative to the clutch member 14 and/or to prevent/limit movement of the scope 66 relative to the clutch member 14 in a direction normal to the clutch axis 16. The displaceable adjustment member 12 coupled to the clutch member 14 may control the degree of engagement between the portions of the proximal engagement members 142 that are configured to selectively engage corresponding portions of the exterior surface of the scope 66 to allow the scope to be displaced within the interior portion 24 to a precise position, and then be locked in that position.

Thus, the scope clutch assembly 10 is configured to receive a variety of scopes 66 of varying diameters and allow for the seamless insertion, rotation, and axial movement of these scopes 66 when disengaged or partially engaged, and provides an ergonomic locking mechanism to maintain exact positioning and stability of the scope 66 during critical surgical steps. The scope clutch assembly 10 thereby enhances procedural precision, minimizes disruptions, and supports a wider range of endoscopic maneuvers, ultimately advancing the efficacy and safety of minimally invasive surgical procedures.

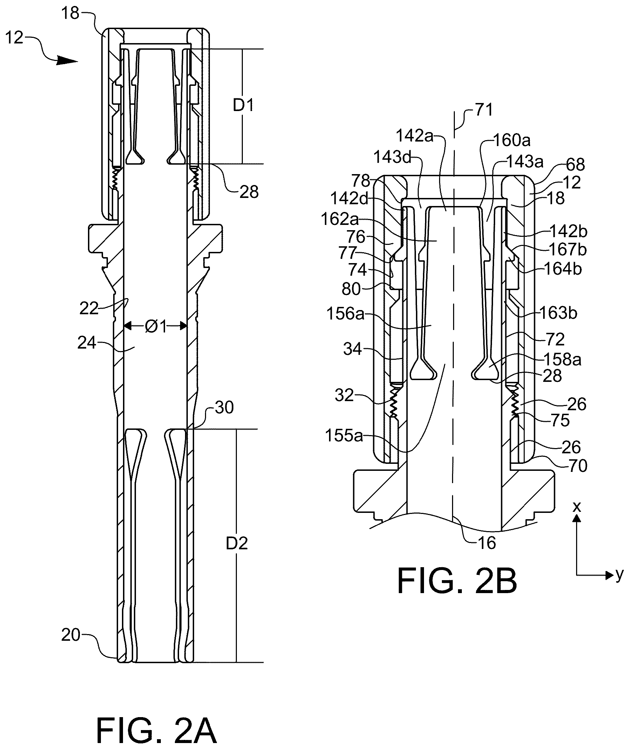

Turing to the clutch member 14 of the scope clutch assembly 10 in more detail, and as illustrated in the cross-sectional view of the scope clutch assembly 10 in Fig. 2A, the clutch member 14 may have one or more internal surfaces 22 that extend from the open proximal end 18 to the open distal end 20, and the one or more internal surfaces 22 may define or cooperate to define an interior portion 24 of the clutch member 14. In the embodiment of Fig. 2A, the one or more internal surfaces 22 may be a single cylindrical surface having a diameter *d*1. However, the one or more internal surfaces 22 may include any number of surfaces that cooperate to form any suitable shape or combination of shapes.

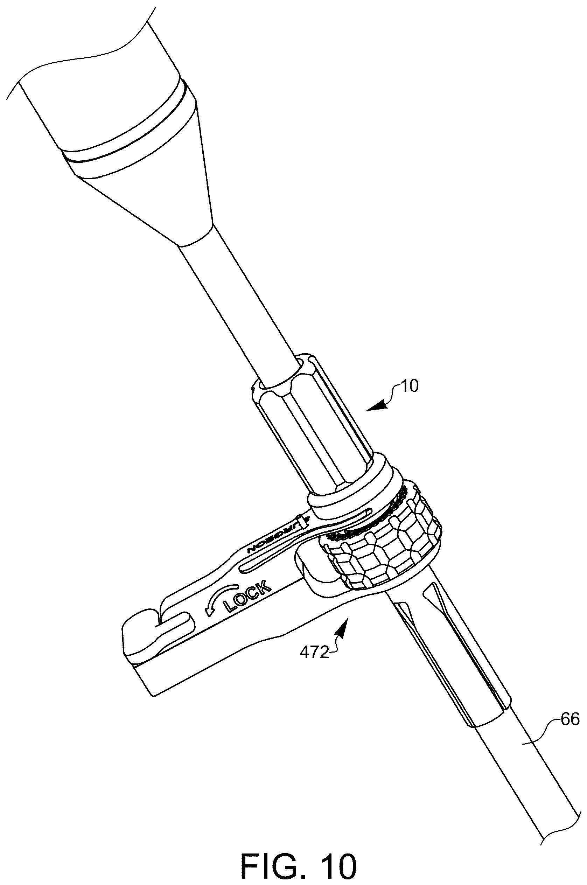

Referring again to Fig. 2A, the clutch member 14 may include the hub portion 26 that extends from a proximal end 28 to a distal end 30. The proximal end 28 of the hub portion 26 may be offset from the proximal end 18 of the clutch member 14 (along the clutch axis 16) by a first distance D1, and the distal end 30 of the hub portion 26 may be offset from the distal end 20 of the clutch member 14 (along the clutch axis 16) by a second distance D2. With reference to Fig. 3A, the hub portion 26 may include an external threaded portion 32 that may be formed on an exterior surface 34 of the clutch member 14 (or of the hub portion 26 of the clutch portion 14), and the external threaded portion 32 may be disposed at or adjacent to the proximal end 28 of the hub portion 26. An annular ridge portion 36 may radially extend from a portion of the exterior surface 34 distal to the external threaded portion 32, a frustoconical portion 38 may radially extend from a portion of the exterior surface 34 distal to the external threaded portion 32 distal to the ridge portion 36. Further, a nut portion 40 may radially extend from a portion of the exterior surface 34 distal to the frustoconical portion 3, and the nut portion 40 may be proximal to the distal end 30 of the hub portion 26. The nut portion 40 may have a plurality of faceted surfaces that are adapted to be engaged by a device to rotate the scope clutch assembly 10 about the clutch axis 16. The nut portion 40 may be configured to engage portions of an adapter to attach the scope clutch assembly 10 to a structure for support, such as the instrument adapter 472 illustrated in Figure 10 and disclosed in International Publication No. WO 2018/204937 (International Application No. PCT/US2018/033288, filed May 17, 2018), which is incorporated by reference herein in its entirety.

A plurality of elongated distal engagement members 42 may extend from the distal end 30 of the hub portion 26. In the embodiment of Fig. 3A, four distal engagement members 42 may extend distally from the hub portion 26 (i.e., a first distal engagement member 42a, a second distal engagement member 42b, a third distal engagement member 42c, and a fourth distal engagement member 42d). Each of the first, second, third and fourth distal engagement members 42a-42d may be at least partially defined by two or more engagement slots 43 that may be symmetrically or asymmetrically arrayed about the clutch member 14. For example, and as illustrated in Fig. 1D, the clutch member 14 may include four engagement slots 43 (i.e., a first engagement slot 43a, a second engagement slot 43b, a third engagement slot 43c, and a fourth engagement slot 43d). Thus, the first engagement slot 43a may be disposed between the first distal engagement member 42a and the second distal engagement member 42b, the second engagement slot 43b may be disposed between the second distal engagement member 42b and the third distal engagement member 42c, the third engagement slot 43c may be disposed between the third distal engagement member 42c and the fourth distal engagement member 42d, and the fourth engagement slot 43d may be disposed between the fourth distal engagement member 42d and the first distal engagement member 42a.

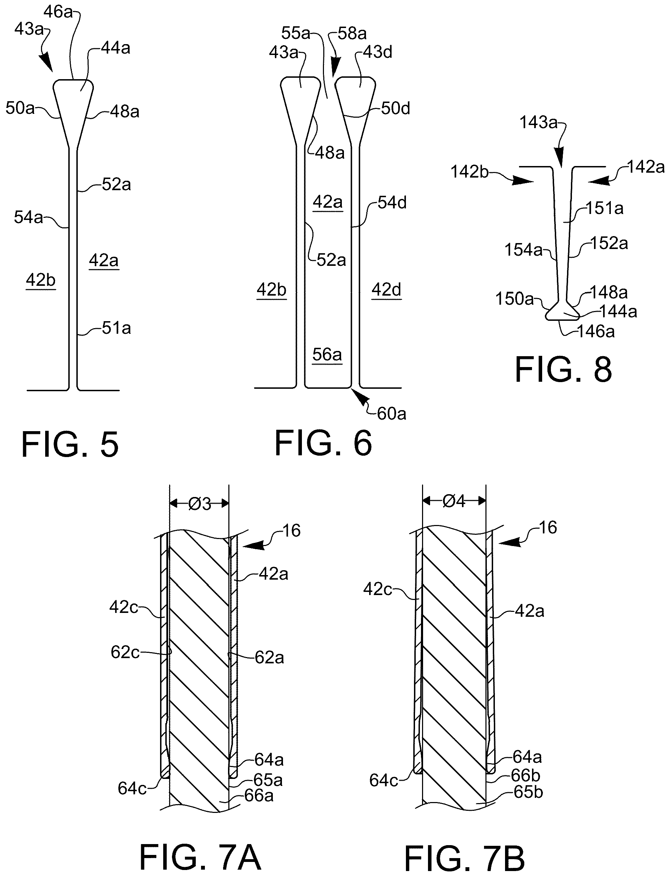

Each of the engagement slots 43 may be identical and arrayed about the clutch axis 16 by equal radial distances, such as in 90° increments. With reference to Fig. 5, which illustrates the first engagement slot 43a (that may be identical to each of the engagement slots 43), the first engagement slot 43a may include a first proximal portion 44a that may be defined by one or more edges, such as a transverse edge 46a that may extend circumferentially about the one or more internal surfaces 22 and may be normal to the clutch axis 16 when viewed along the Y-Z plane of the reference coordinate system of Figs. 1A and 1C. The transverse edge 46a may be at or adjacent to the distal end 30 of the hub portion 26. The first proximal portion 44a may be further defined by a first oblique edge 48a having a first end that extends from a first end of the transverse edge 46a and a second oblique edge 50a having a first end that extends from a second end of the transverse edge 46a, and each of the first oblique edge 48a and the second oblique edge 48a converge symmetrically as the first oblique edge 48a and the second oblique edge 48a extend distally. However, a second end of the first oblique edge 46a and a second end of the second oblique edge 48a are circumferentially offset and do not intersect. So configured, the first proximal portion 44a has the general shape of an inverted triangle. In some embodiments, the intersections of the transverse edge 46a and the first oblique edge 48a and the second oblique edge 48a may be radiused or rounded.

The first segment slot 43a may include a first distal portion 51a which may extend distally from the first proximal portion 44a to the distal end 20 of the clutch member 14. The first distal portion 51a may be defined by a first lateral edge 52a and a second lateral edge 54a. The first lateral edge 52a may extend from the second end of first oblique edge 48a to the distal end of the clutch member 14, and the first lateral edge 52a may extend in a direction that is parallel to the X-axis of the reference coordinate system of Figs. 1A. However, the first lateral edge 52a may have any shape or combination of shapes that may generally extend in a direction that is parallel to the X-axis of the reference coordinate system of Fig. 3A. The second lateral edge 54a may extend from the second end of second oblique edge 50a to the distal end of the clutch member 14, and the second lateral edge 54a may be parallel to the first lateral edge 52a and extend in a direction that is parallel to the X-axis of the reference coordinate system of Figs. 1A. However, the second lateral edge 54a may have any shape or combination of shapes that may generally extend in a direction that is parallel to the X-axis of the reference coordinate system of Fig. 3A. The first lateral edge 52a may be circumferentially offset from the second lateral edge 54a by a distance that is between 5% to 50% (e.g., between 5% to 20%) of the length of the transverse edge 46a and may form an angle between 3° and 20° (e.g., between 5° and 15°) when viewed along the clutch axis 16.

As previously explained, each of the second, third, and fourth engagement slots 43b-43d may be identical to the first engagement slot 43a, and features of the second, third, and fourth engagement slots 43b-43d will be identical to the corresponding features of the first engagement slot 43a and will be indicated by a "b", "c", or "d" at the end of the corresponding reference number. For example, the second engagement slot 43b may include a second proximal portion 44b, the third engagement slot 43c may include a third proximal portion 44c, and the fourth engagement slot 43d may include a fourth proximal portion 44d that may be each be identical in shape and function to the first proximal portion 44a of the first engagement slot 43a.

As previously explained, each of the plurality of elongated distal engagement members 42 may be defined by one or more portions of each of the two adjacent engagement slots 43. In particular, as illustrated in Fig. 6, the first distal engagement member 42a may extend from a proximal end 58a that is at a distal end 30 of the hub portion 26 to a distal end 60a that is at the distal end 20 of the clutch member 14. The first distal engagement member 42a may have a proximal portion 55a that may be partially defined by the first oblique edge 48a of the first engagement slot 43a and the second oblique edge 50d of the of the fourth engagement slot 43d. So configured, the first oblique edge 48a of the first engagement slot 43a and the second oblique edge 50d of the of the fourth engagement slot 43d diverge symmetrically as the first oblique edge 48a and the second oblique edge 50d extend distally. In addition, the first distal engagement member 42a may have a distal portion 56a that may be partially defined by the first lateral edge 52a of the first engagement slot 43a and the second lateral edge 54d of the of the fourth engagement slot 43d, and each of the first lateral edge 52a of the first engagement slot 43a and the second lateral edge 54d of the of the fourth engagement slot 43d extend distally from the second end of the first oblique edge 48a and the second end of the second oblique edge 50d, respectively, to the distal end 30 of the clutch member 14. Each of the first lateral edge 52a of the first engagement slot 43a and the second lateral edge 54d of the of the fourth engagement slot 43d extend parallel or substantially parallel to the X-axis of the reference coordinate system of Fig. 3A. However, in some embodiments, the first lateral edge 52a of the first engagement slot 43a and the second lateral edge 54d of the of the fourth engagement slot 43d may converge (or diverge) as the first lateral edge and the second lateral edge 54d extend distally from the second end of the first oblique edge 48a and the second end of the second oblique edge 50d, respectively. So configured, the first distal engagement member 42a may act as a cantilevered leaf spring such that if a force (that is directed along the Z-axis of the reference coordinate system of Fig. 1B) is applied to the first distal engagement member 42a, a portion of the first distal engagement member 42a at the distal end 60a will displace (along the Z-axis of the reference coordinate system of Fig. 1B) relative to a portion of the first distal engagement member 42a at the proximal end 58a.

As illustrated in the cross-sectional view of the first distal engagement member 42a of Fig. 4, the first distal engagement member 42a may be partially defined by a first interior surface 62a that may be a portion of the one or more internal surfaces 22 that define the interior portion 24 of the clutch member 14, and all or a portion of the first interior surface 62a may have the shape of a portion of a cylinder that may have the diameter *d*1. In some embodiments, a portion of the first interior surface 62a may have a reduced diameter portion between the proximal end 58a and the distal end 60a. An engagement protrusion 64a may be disposed on or along a portion of the first interior surface 62a, and the engagement protrusion 64a may extend radially inwards towards the clutch axis 16. The engagement protrusion 64a may extend circumferentially along the first interior surface 62a from the first lateral edge 52a of the first engagement slot 43a to the second lateral edge 54d of the of the fourth engagement slot 43d, or may extend circumferentially along one or more portions of the first interior surface 62a from the first lateral edge 52a of the first engagement slot 43a to the second lateral edge 54d of the of the fourth engagement slot 43d. The engagement protrusion 64a may have a uniform cross-sectional shape along the circumferential length of the engagement protrusion 64a (or substantially the circumferential length of the engagement protrusion 64a), or one of more circumferential portions of the engagement protrusion 64a may have different cross-sectional shapes along the circumferential length of the engagement protrusion 64a. In addition, the engagement protrusion 64a may extend circumferentially along a portion of the first interior surface 62a that is at or adjacent to the distal end 60a of the first distal engagement member 42a.

The engagement protrusion 64a may have any suitable shape or combination of shapes that are configured to engage a portion of a scope that is inserted into the interior portion 24 of the clutch member 16. For example, as illustrated in Fig. 4, the engagement protrusion 64a may have the shape of a rounded, sloped protrusion extending away from the first interior surface 62a. However, the engagement protrusion 64a may have any suitable shape or combination of shapes configured to engage or contact a portion of an exterior surface 65a of a first scope 66a (as illustrated in Fig. 7A) and a portion of an exterior surface 65b of a second scope 66b (as illustrated in Fig. 7B), as will be described in more detail below. As illustrated in Fig. 4, an apex of the engagement protrusion 64a of the first distal engagement member 42a may be separated from an apex of the engagement protrusion 64c of the first distal engagement member 42c by a distance *d*2, and because of the uniform cross-sectional shape of the engagement protrusions 64a-64d, the apexes of the engagement protrusions 64a-64d define a diameter *d*2, which may be equal or substantially equal to diameter *d*1. In other embodiments, diameter *d*2 may be smaller (e.g. 3% to 10% smaller) that diameter *d*1. Thus, when the first scope 66a is inserted into the interior portion 24 of the clutch member 14 and advanced until a portion of the first scope 66a extends beyond the distal end 20 of the clutch member, and when the diameter of the first scope 66a has a diameter *d*3 (e.g., 10mm) that is equal to or slightly larger than diameter *d*2, the apexes of the engagement protrusions 64a-64d of the distal engagement members 42a-42d are in engagement with corresponding portions of the exterior surface 65a of the first scope 66a such that movement (or shaking) of the first scope 66a relative to the clutch member 14 is reduced or prevented. Thus, by reducing or eliminating movement of the first scope 66a, images captured by the first scope 66a will be clear and focused.

However, as illustrated in Fig. 7B, the scope clutch assembly 10 may also receive the second scope 66b, which has a diameter *d*4 (e.g., 10.3 mm) that is larger than diameter *d*3 and diameter *d*2, but less than or equal to diameter *d*1. Thus, when the second scope 66b is inserted into the interior portion 24 of the clutch member 14 and advanced until a portion of the second scope 66b extends to the distal end 20 of the clutch member 14, the apexes of the engagement protrusions 64a-64d of the distal engagement members 42a-42d come into engagement with corresponding portions of the exterior surface 65b of the second scope 66b. Because the diameter *d*4 is greater than diameter *d*2, the distal ends 60a-60d of the distal engagement members 42a-42d are forced outwards due to the cantilevered spring properties of the distal engagement members 42a-42d to maintain sealing engagement between the engagement protrusions 64a-64d of the distal engagement members 42a-42d and the corresponding portions of the exterior surface 65a of the first scope 66a. Thus, movement (or shaking) of the second scope 66b relative to the clutch member 14 is reduced or prevented. Accordingly, the single scope clutch assembly 10 may reduce or eliminate shaking of both the first scope 66a and a differently sized second scope 66b (and any other scope having a diameter between diameter *d*1 and diameter *d*2), thereby eliminating the need for a different mounting assembly for each scope.

The clutch member 14 may also include a plurality of elongated proximal engagement members 142 that may extend from the proximal end 28 of the hub portion 26 such that each of the plurality of elongated proximal engagement members 142 may extend along or parallel to the clutch axis 16 by the first distance D1, as illustrated in Fig. 2A.

In the embodiment of Fig. 3A, four proximal engagement members 142 may extend proximally from the hub portion 26 (i.e., a first proximal engagement member 142a, a second proximal engagement member 142b, a third proximal engagement member 142c, and a fourth proximal engagement member 142d). Each of the first to fourth proximal engagement members 142a-142d may be similar to or substantially identical in shape to the corresponding first distal engagement member 142a, the second distal engagement member 42b, the third distal engagement member 42c, and the fourth distal engagement member 42d, respectively. That is, each of the first, second, third and fourth proximal engagement members 142a-142d may be at least partially defined by two or more engagement slots 143 that may be symmetrically or asymmetrically arrayed about the clutch member 14. For example, and as illustrated in Fig. 3B, the clutch member 14 may include four engagement slots 143 (i.e., a first engagement slot 143a, a second engagement slot 143b, a third engagement slot 143c, and a fourth engagement slot 143d), that may be similar or identical to the engagement slots 43a-43d previously described. Thus, the first engagement slot 143a may be disposed between the first proximal engagement member 142a and the second proximal engagement member 142b, the second engagement slot 143b may be disposed between the second proximal engagement member 142b and the third proximal engagement member 142c, the third engagement slot 143c may be disposed between the third proximal engagement member 142c and the fourth proximal 1 engagement member 142d, and the fourth engagement slot 143d may be disposed between the fourth proximal engagement member 142d and the first proximal engagement member 142a.

Each of the engagement slots 143 may be identical and arrayed about the clutch axis 16 by equal radial distances, such as in 90° increments. With reference to Fig. 8, which illustrates the first engagement slot 143a (that may be identical to each of the engagement slots 143), the first engagement slot 143a may include a first distal portion 144a that may be defined by one or more edges, such as a transverse edge 146a that may extend circumferentially about the one or more internal surfaces 22 and may be normal to the clutch axis 16 when viewed along the Y-Z plane of the reference coordinate system of Figs. 1A and 1C. The transverse edge 146a may be at or adjacent to the proximal end 28 of the hub portion 26. The first distal portion 44a may be further defined by a first oblique edge 148a having a first end that extends from a first end of the transverse edge 146a and a second oblique edge 150a having a first end that extends from a second end of the transverse edge 146a, and each of the first oblique edge 148a and the second oblique edge 148a converge symmetrically as the first oblique edge 148a and the second oblique edge 148a extend proximally. However, a second end of the first oblique edge 146a and a second end of the second oblique edge 148a are circumferentially offset and do not intersect. So configured, the first distal portion 144a has the general shape of a triangle. In some embodiments, the intersections of the transverse edge 146a and the first oblique edge 148a and the second oblique edge 148a may be radiused or rounded.

The first segment slot 143a may include a first proximal portion 151a which may extend proximally from the first distal portion 144a to the proximal end 28 of the clutch member 14. The first proximal portion 151a may be defined by a first lateral edge 152a and a second lateral edge 154a. The first lateral edge 152a may extend from the second end of first oblique edge 148a to the distal end of the clutch member 14. The second lateral edge 154a may extend from the second end of second oblique edge 150a to the proximal end 28 of the clutch member 14, and the second lateral edge 154a. Each of the first lateral edge 152a and the second lateral edge 154a may diverge symmetrically as the first lateral edge 152a and the second lateral edge 154a extend proximally. However, in some embodiments, each of the first lateral edge 152a and the second lateral edge 154a may be parallel and extend in a direction that is parallel to the X-axis of the reference coordinate system of Figs. 1A.

As previously explained, each of the second, third, and fourth engagement slots 143b-143d may be identical to the first engagement slot 143a, and features of the second, third, and fourth engagement slots 143b-143d will be identical to the corresponding features of the first engagement slot 143a and will be indicated by a "b", "c", or "d" at the end of the corresponding reference number. For example, the second engagement slot 143b may include a second proximal portion 151b, the third engagement slot 143c may include a third proximal portion 151c, and the fourth engagement slot 143d may include a fourth proximal portion 151d that may be each be identical in shape and function to the first proximal portion 151a of the first engagement slot 143a.

As previously explained, each of the plurality of elongated proximal engagement members 142 may be defined by one or more portions of each of the two adjacent engagement slots 143. In particular, and with reference to Fig. 2B, the first distal engagement member 142a may extend from a distal end 158a that is at a proximal end 28 of the hub portion 26 to a proximal end 160a that is at the proximal end 18 of the clutch member 14. The first proximal engagement member 142a may have a distal portion 155a that may be partially defined by the first oblique edge 148a of the first engagement slot 43a and the second oblique edge 150d of the of the fourth engagement slot 143d. So configured, the first oblique edge 148a of the first engagement slot 143a and the second oblique edge 150d of the of the fourth engagement slot 143d diverge symmetrically as the first oblique edge 48a and the second oblique edge 50d extend proximally. In addition, the first proximal engagement member 142a may have a proximal portion 156a that may be partially defined by the first lateral edge 152a of the first engagement slot 143a and the second lateral edge 154d of the of the fourth engagement slot 143d, and each of the first lateral edge 152a of the first engagement slot 143a and the second lateral edge 154d of the of the fourth engagement slot 143d extend distally from the second end of the first oblique edge 148a and the second end of the second oblique edge 150d, respectively, to the proximal end 28 of the clutch member 14. Each of the first lateral edge 152a of the first engagement slot 143a and the second lateral edge 154d of the of the fourth engagement slot 143d may converge as the first lateral edge 152d and the second lateral edge 154d extend proximally from the second end of the first oblique edge 148a and the second end of the second oblique edge 150d, respectively. So configured, the first proximal engagement member 142a may act as a cantilevered leaf spring such that if a force (that is directed along the Z-axis of the reference coordinate system of Fig. 1B) is applied to the first proximal engagement member 142a (at or adjacent to the proximal end 160a), a portion of the first proximal engagement member 142a at the proximal end 160a will displace (along the Z-axis of the reference coordinate system of Fig. 1B) relative to a portion of the first proximal engagement member 142a at the distal end 158a.

The first proximal engagement member 142a may be partially defined by a first interior surface 162a that may be a portion of the interior portion 24 of the clutch member 14, and all or a portion of the first interior surface 162a may have the shape of a portion of a cylinder that may have the diameter *d*1.

As illustrated in the cross-sectional view of the second proximal engagement member 142b of Fig. 2B, the second proximal engagement member 142b may be partially defined by a second exterior surface 163b that may be a portion of the exterior surface 34 of the clutch member 14, and all or a portion of the second exterior surface 163b may have the shape of a portion of a cylinder. An engagement protrusion 164b may be disposed on or along a portion of the second exterior surface 163b, and the engagement protrusion 164b may extend radially outwards away from the clutch axis 16. The engagement protrusion 164b may extend circumferentially along the second exterior surface 163b from the first lateral edge 152b of the second engagement slot 143b to the second lateral edge 54c of the of the third engagement slot 43c, or may extend circumferentially along one or more portions of the second exterior surface 163b. The engagement protrusion 164b may have a uniform cross-sectional shape along the circumferential length of the engagement protrusion 164b (or substantially the circumferential length of the engagement protrusion 164b), or one of more circumferential portions of the engagement protrusion 164b may have different cross-sectional shapes along the circumferential length of the engagement protrusion 164b. In addition, the engagement protrusion 164b may extend circumferentially along a portion of the second exterior surface 163b that adjacent to or distally offset from the proximal end 160b of the second distal engagement member 142b.

The engagement protrusion 164b may have any suitable shape or combination of shapes that are configured to engage a portion of the adjustment member 12 to tighten or loosen the four proximal engagement members 142a-142d in a manner that will be described below. For example, the may have a frustoconical contact surface 167b.

As illustrated in Fig. 1A, the scope clutch assembly 10 may include the adjustment member 12 that may be displaceably coupled to a portion of the clutch member 14. With reference to the cross-sectional view of Fig. 2B of the scope clutch assembly 10, the adjustment member 12 may extend from a proximal end 68 to a distal end 70 along a member axis 71 that may be coaxially aligned with the clutch axis 16. The adjustment member 12 may have a central aperture 72 that may extend along or substantially along the member axis 71, and the central aperture 72 may be defined by one or more internal surfaces 74. A first portion of the one or more internal surfaces 74 may include a threaded portion 75 that may be configured to engage the external threaded portion 32 of the hub portion 26. So configured, a rotation of the adjustment member 12 about the member axis 71 in a first direction causes the adjustment member 12 to displace distally along the member axis 71 relative to the clutch member 14, and a rotation of the adjustment member 12 about the member axis 71 in a second direction causes the adjustment member 12 to displace proximally along the member axis 71 relative to the clutch member 14.

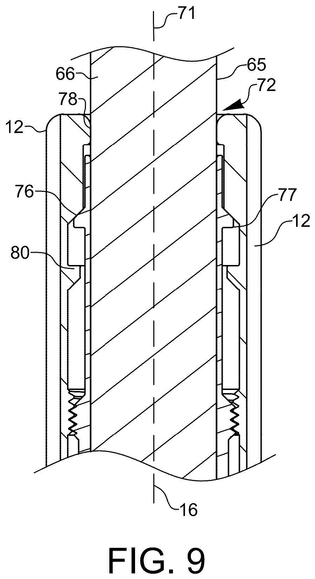

Still referring to Fig. 2B, a second portion of the one or more internal surfaces 74 of the adjustment member 12 may include a circumferential ridge portion 76 that projects inward towards the member axis 71. In some embodiments, the ridge portion 76 may extend along the entire circumference of the one or more internal surfaces 74 and may have a uniform cross-sectional shape along the entire circumference. The ridge portion 76 may include a frustoconical contact surface 77 that may be configured to contact, mate with, and/or engage and mate with the frustoconical contact surface 167b of the engagement protrusion 164v of the clutch member 14. For example, in a first adjustment position, the contact surface 77 of the ridge portion 76 may be proximally offset from the contact surface 167b of the engagement protrusion 164b. When the adjustment member 12 is distally displaced into a second adjustment position (illustrated in Fig. 2B), the contact surface 77 of the ridge portion 76 may come into contact with the contact surface 167b of the engagement protrusion 164. Due to the complementary oblique cross-sectional shapes of the contact surface 77 of the ridge portion 76 and the contact surface 167b of the engagement protrusion 164b, further distal displacement of the adjustment member 12 relative to the clutch member 14 into a third adjustment position (illustrated in Fig. 9, in which the scope 66 is included) forces the engagement protrusion 164b, and the proximal end 160b of the first proximal engagement member 142b, inwards towards the clutch axis 16. Because all of the contact surfaces 167a-167d of the engagement protrusions 164a-164d are contacted in the same manner by corresponding portions of the contact surface 77 of the ridge portion 76, the proximal ends 160, 160c, 160d of each of the first, third, and fourth proximal engagement members 142b-142d are displaced towards the member axis 71 in the third position in the same manner as the proximal end 160b of the second proximal engagement member 142b.

In this third adjustment position, a portion of the second interior surface 162b of the second proximal engagement member 142b may contact a corresponding potion of the exterior surface 65 of scope 66, and a portion of each of the second interior surfaces 162a, 162c, 162d of the first, third, and fourth proximal engagement members 142b-142d may contact corresponding potions of the exterior surface 65 of scope 66. Thus, in the third position, the first to fourth proximal engagement members 142a-142d cooperate to secure the corresponding potion of the exterior surface 65 of scope 66 to (a) prevent the scope 66 from displacing along the clutch axis 16 relative to the clutch member 14 and/or (b) to prevent/limit movement of the scope 66 relative to the clutch member 14 in a direction normal to the clutch axis 16 (at or adjacent to the proximal ends 160a-160d of the first to fourth proximal engagement members 142a-142d) to stabilize the scope 66. To loosen the first to fourth proximal engagement members 142b-142d to allow the scope 66 to be longitudinally displaced along the clutch axis 16, the adjustment member 12 may be displaced along the member axis 71 from the third adjustment position to the first adjustment position.

The adjustment member 12 may also include a lip portion 78 at or adjacent to the proximal end 68 that is configured to engage the proximal ends 160a-160d the first to fourth proximal engagement members 142a-142d to prevent or limit further distal displacement of the adjustment member 12 relative to the clutch member 14. Further, a third portion of the one or more internal surfaces 74 of the adjustment member 12 may define a circumferential stop portion 80 that projects inward towards the member axis 71. The stop portion 80 may extend along the entire circumference of the one or more internal surfaces 74 and may have a uniform cross-sectional shape. The stop portion 80 may be configured to contact a portion of the engagement protrusions 164b on any or all of the first to fourth proximal engagement members 142a-142d to prevent or limit further proximal displacement of the adjustment member 12 relative to the clutch member 14. Thus, by proximally displacing the adjustment member 12 to a point in which the threaded portion 75 is displaced proximally out of engagement with the external threaded portion 32 of the clutch member 14, the adjustment member 12 may freely rotate relative to the clutch member 14, and contact between the stop 80 and any or all of the engagement protrusions 164a-164d of the clutch member 14 may prevent the adjustment member 12 from being removed from the clutch member 14. With the threaded portion 75 of the adjustment member 12 out of threaded engagement with the external threaded portion 32 of the clutch member 14, the scope clutch assembly 10 may be sterilized without potential contaminants being trapped between engaged threads.

The clutch member 14 and the adjustment member 12 may each be fabricated or manufactured from any suitable material. In some embodiments, the clutch member 14 and the adjustment member 12 are each made from a metal material, and the scope clutch assembly 10 may be sterilizable and re-usable. For example, the clutch member 14 may be fabricated or manufactured from SS 17-4 H900 and the adjustment member 12 may be fabricated or manufactured from Nitronic 60. All or a portion of the clutch member 14 may be made from (or coated with) materials with high frictional grip. For example, all or a portion of the engagement protrusions 64a and/or engagement protrusion 164b may be made from, or coated, with high frictional grip materials.

Various advantages of a scope clutch assembly have been discussed above. Embodiments discussed herein have been described by way of example in this specification. It will be apparent to those skilled in the art that the foregoing detailed disclosure is intended to be presented by way of example only, and is not limiting. As just one example, although the end effectors in the discussed examples were often focused on the use of a scope, such systems could be used to position other types of surgical equipment. Various alterations, improvements, and modifications will occur and are intended to those skilled in the art, though not expressly stated herein. These alterations, improvements, and modifications are intended to be suggested hereby, and are within the scope of the claimed invention. The drawings included herein are not necessarily drawn to scale. Additionally, the recited order of processing elements or sequences, or the use of numbers, letters, or other designations therefore, is not intended to limit the claims to any order, except as may be specified in the claims. Accordingly, the invention is limited only by the following claims and equivalents thereto.

## Claims

1. A clutch assembly (10) comprising:
a clutch member (14) extending along a clutch axis (16) from a proximal end (18, 28, 58a, 68, 160a, 160b) to a distal end (20, 30, 60a, 70, 158a), the clutch member (14) having one or more interior surfaces that define an interior portion (24), the clutch member (14) comprising:
a hub portion (26) extending from a proximal end (18, 28, 58a, 68, 160a, 160b) to a distal end (20, 30, 60a, 70, 158a), the proximal end (18, 28, 58a, 68, 160a, 160b) of the hub portion (26) being offset from the proximal end (18, 28, 58a, 68, 160a, 160b) of the clutch member (14) along the clutch axis (16) by a first distance, and the distal end (20, 30, 60a, 70, 158a) of the hub portion (26) may be offset from the distal end (20, 30, 60a, 70, 158a) of the clutch member (14) along the clutch axis (16) by a second distance, the hub portion (26) having an external threaded portion (32) formed on a portion of an exterior surface (34, 65, 65a, 65b) of the hub portion (26);
a plurality of elongated distal engagement members (42), each of the plurality of distal engagement members (42) extending from a proximal end (18, 28, 58a, 68, 160a, 160b) to a distal end (20, 30, 60a, 70, 158a), the proximal end (18, 28, 58a, 68, 160a, 160b) of each of the plurality of distal engagement members (42) extending from a corresponding portion of the distal end (20, 30, 60a, 70, 158a) of the hub portion (26) , and the distal end (20, 30, 60a, 70, 158a) of at least one of the plurality of distal engagement members (42) being disposed at the distal end (20, 30, 60a, 70, 158a) of the clutch member (14);
a plurality of elongated proximal engagement members (142), each of the plurality of proximal engagement members (142) extending from a proximal end (18, 28, 58a, 68, 160a, 160b) to a distal end (20, 30, 60a, 70, 158a), the distal end (20, 30, 60a, 70, 158a) of each of the plurality of proximal engagement members (142) extending from a corresponding portion of the proximal end (18, 28, 58a, 68, 160a, 160b) of the hub portion (26), and the proximal end (18, 28, 58a, 68, 160a, 160b) of at least one of the plurality of proximal engagement members (142) being disposed at the proximal end (18, 28, 58a, 68, 160a, 160b) of the clutch member (14), wherein a proximal engagement protrusion (64a, 64c, 164, 164b, 164v) is disposed on or along a portion of an exterior surface (34, 65, 65a, 65b) of each of the plurality of proximal engagement members (142), and wherein each of the proximal engagement protrusions (64a) 64a extends radially outward from the clutch axis (16); and
an adjustment member (12) coupled to the clutch member (14), the adjustment member (12) extending from a proximal end (18, 28, 58a, 68, 160a, 160b) to a distal end (20, 30, 60a, 70, 158a) along a member axis (71) that may be coaxially aligned with the clutch axis (16) , the adjustment member (12) having a central aperture (72) extending along the member axis (71), and the central aperture (72) being defined by one or more internal surfaces (22, 74), with a first portion of the one or more internal surfaces (22, 74) including a threaded portion (75) that is configured to engage the external threaded portion (32) formed on the exterior surface (34, 65, 65a, 65b) of the hub portion (26) such that the adjustment member (12) is displaceable relative to the clutch member (14), and with a second portion of the one or more internal surfaces (22, 74) defining a circumferential ridge portion (76);
wherein a portion of a contact surface (77, 167b) of the ridge portion (36, 76) is configured to engage a corresponding contact surface (77, 167b) of one of the proximal engagement protrusions (64a) such that
(a) when the adjustment member (12) is displaced in a first direction relative to the clutch member (14) along the member axis (71), the proximal end (18, 28, 58a, 68, 160a, 160b) of each of the proximal engagement protrusions (64a) is displaced towards the member axis (71) to increase engagement between the proximal engagement protrusions (64a) and corresponding portions of an exterior surface (34, 65, 65a, 65b) of a scope (66) such that displacement of the scope (66) relative to the clutch member (14) is prevented; and
(b) when the adjustment member (12) is displaced in a second direction relative to the clutch member (14) along the member axis (71), the proximal end (18, 28, 58a, 68, 160a, 160b) of each of the proximal engagement protrusions (64a) is displaced away from the member axis (71) to decrease engagement between the proximal engagement protrusions (64a) and the corresponding portions of the exterior surface (34, 65, 65a, 65b) of the scope (66) such that displacement of the scope (66) relative to the clutch member (14) is allowed.

2. The clutch assembly (10) of claim 1, wherein a distal engagement protrusion (64a, 64c, 164, 164b, 164v) is disposed on or along a portion of a first interior surface (62a, 162a) of each of the plurality of distal engagement members (42), with each of the first interior surfaces comprising a portion of the one or more interior surfaces that define the interior portion (24);
wherein each of the distal engagement protrusions (64a, 164b) 64a extends radially inwards towards the clutch axis (16), and
wherein each of the distal engagement protrusions (64a, 164b) configured to engage corresponding portions of the exterior surface (34, 65, 65a, 65b) of the scope (66) such that shaking of the scope (66) relative to the clutch member (14) is reduced.

3. The clutch assembly (10) of claim 2, wherein the portion of the first interior surface (62a, 162a) of each of the plurality of distal engagement members (42) is at or adjacent to the distal end (20, 30, 60a, 70, 158a) of each of the distal engagement members (42).

4. The clutch assembly (10) of any one of claims 1 to 3, wherein each of the distal engagement members (42) inwardly converges towards the clutch axis (16) from the proximal end (18, 28, 58a, 68, 160a, 160b) to the distal end (20, 30, 60a, 70, 158a) such that the engagement protrusion (64a, 64c, 164, 164b, 164v) of each of the distal engagement members (42) is configured to be biased into engagement with the corresponding portions of the exterior surface (34, 65, 65a, 65b) of the scope (66).

5. The clutch assembly (10) of any one of claims 1 to 4, wherein each of the plurality of proximal engagement members (142) is at least partially defined by two or more engagement slots (43, 143) symmetrically arrayed about the clutch member (14).

6. The clutch assembly (10) of any one of claims 1 to 5, wherein four distal engagement members (42) extend distally from the hub portion (26).

7. The clutch assembly (10) of claim 5 or 6, wherein each of the engagement slots (43, 143) are arrayed about the clutch axis (16) in 90° increments.

8. The clutch assembly (10) of any one of claims 1 to 7, wherein a nut portion (40) radially extends from a second portion of the exterior surface (34, 65, 65a, 65b) of the hub portion (26), the nut portion (40) having a plurality of faceted surfaces that are adapted to be engaged by a device to rotate the clutch assembly about the clutch axis (16).

9. The clutch assembly (10) of any one of claims 1 to 8, wherein the external threaded portion (32) is disposed at or adjacent to the proximal end (18, 28, 58a, 68, 160a, 160b) of the hub portion (26).
